# EUROPEAN PATENT APPLICATION

(11) **EP 4 005 466 A1**
(43) Date of publication of application: **01.06.2022**
(21) Application number: 21206351.5
(22) Date of filing: 04.11.2021
(51) Int. Cl.: A61B 5/00, A61B 5/11, G16H 50/20, G16H 50/70, G16H 80/00, G08B 21/04

(54) **SAFETY SYSTEM AND METHOD FOR MONITORING NOT SELF-SUFFICIENT PERSONS**

(30) Priority: 27.11.2020 IT 202000028880
(71) Applicant: Dom-Ino Labs S.r.l., 88100 Catanzaro (CZ) (IT)
(72) Inventor: RICCI, Luciano, 88100 Catanzaro (CZ) (IT)
(74) Representative: Giuliano, Natalia

(57) **Abstract**

Safety system (100) for monitoring not self-sufficient persons, comprising:
- at least one bed (101) comprising at least one weight sensor (101b);
- a plurality of sensors (101c;
- at least one acoustic sensor (101e);
- at least one thermal camera (101d);
- at least one movement sensor (101f);
- at least one wearable sensor (102);
- at least one sensor (101g) able to remotely monitor vital parameters of the patient from afar.

The safety system (100), in addition, comprises:
- at least one embedded processing unit (101a), provided with a memory unit and with a local database;
- at least one computer (103), connected to the embedded processing unit (101a) and to the Internet, comprising a control system, a central database, a graphical interface and a plurality of algorithms;
- at least one mobile terminal;
- at least one video camera, one microphone and one loudspeaker.

## Description

The present invention relates to a safety system for monitoring not self-sufficient persons.

In particular, the present invention relates to a safety system and method for monitoring patients with cognitive disorders at risk of self-injury due to a fall and/or unconsciousness and/or loss.

Furthermore, the present invention relates to a safety system and method for monitoring patients or persons not self-sufficient and having cognitive disorders, for example patients suffering from diseases such as Alzheimer's or sleepwalking.

It is known how serious the problems caused by Alzheimer's, which is the most common cause of dementia, are. In fact, Alzheimer's represents 60% of cases of dementia and creates, like other neurological degenerative diseases, a state of total dependence of the patient, thus generating a very complex and difficult situation for the family who has to assist the same patient, and also for a health facility having the task of assisting and treating the patient himself. Assistance weighs, for 80% of the cases, on the family which must devote the entire day to caring for the sick, with considerable psychological stress and waste of economic resources. However, even when the patient is entrusted to health facilities, these last see their costs rise, due to the constant monitoring that this disease requires. In fact, it is necessary to prepare resources and personnel day and night, with inevitable technical/economic implications. In addition, sleepwalking risks causing even serious involuntary injuries to those who suffer from it.

Several safety systems and methods for monitoring not self-sufficient persons are currently known.

A first technical solution is disclosed by the European patent application EP3567563A1, whose subject is a system for monitoring patients with cognitive disorders comprising one bed having a plurality of weight sensors, a plurality of sensors configured to detect the descending of the patient from the bed, a plurality of acoustic sensors, a plurality of flame sensors, a plurality of shock sensors, a wearable sensor, a processing unit, a video camera and a computer.

The patent application US2016063846A1, instead, describes a system for determining the location of a patient on a hospital bed comprising a deformation sensor adapted to generate a signal indicative of a deformation of a frame of the bed, and a location determination unit for determining a lateral and/or longitudinal location of the patient based on the deformation of the frame. A weight sensing system for a hospital bed, having a base with a suspended frame suspended from a fixed frame, comprises a load sensor connecting the suspended frame and the fixed frame via a suspension member, which is unsecured from the fixed frame, to allow free vertical movement of the suspended frame relative to the fixed frame.

Still, the patent application WO2014165798 relates to a method and an apparatus for low-cost detection and alerting of bed-departure, which causes significant stress to caretakers of patients who often wander out of bed as a result of their dementia, Alzheimer's disease, or other medical conditions. In one aspect, the apparatus comprises a sock embedded with a pressure sensor and a battery-powered microcontroller unit with a radio frequency module. Once the user wanders out of bed and steps onto the floor, the sensor on the sock will immediately detect the pressure caused by his or her body weight and wirelessly trigger an audible sound at a caretaker's monitoring unit, which can be a smartphone, a tablet or a PC. In one aspect, the sensor and the microcontroller unit can be packaged in one assembly to be mounted on an ordinary sock, slipper, or shoe. The apparatus also accurately counts steps and measures the time interval between them. In one aspect, the pressure sensor and/or the sensor/microcontroller unit assembly can adhere to a foot.

However, such known safety systems are, despite their complexity, unable to cover all the functions necessary to overcome the problems described so far, for instance, they don't provide caregivers and health professionals with advanced data analysis, or predictive and preventive intervention capabilities.

The purpose of the present invention is to provide a safety system for monitoring not self-sufficient persons, which is able to control and monitor the patient in real time in a reliable, fast and efficient manner, optimizing the human resources operating both in the health facility and in the family, having, therefore, characteristics such as to overcome the limits of the current known systems.

According to the present invention, a safety system for monitoring not self-sufficient persons is provided, as defined in claim 1.

According to the present invention, a safety method for monitoring not self-sufficient persons is also provided, as defined in claim 9.

For a better understanding of the present invention, a preferred embodiment is now described, purely by way of nonlimiting example, with reference to the attached drawing, in which:

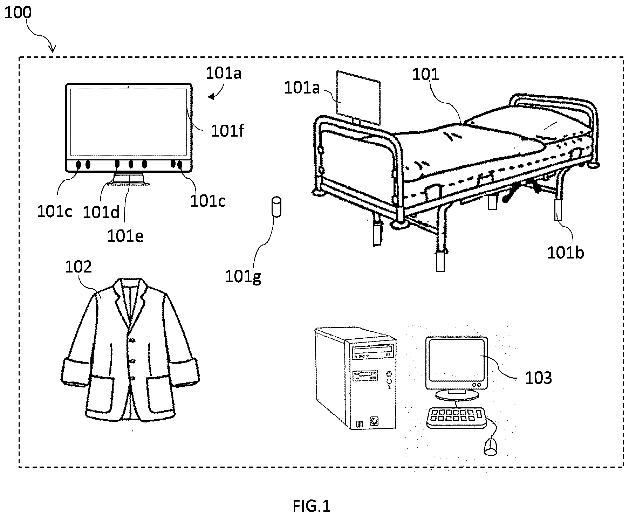

- figure 1 shows a schematic view of a safety system for monitoring not self-sufficient persons, according to the invention.

With reference to figure 1, a safety system 100 for monitoring not self-sufficient persons is shown, according to the invention.

In more detail, the safety system 100 comprises:
- at least one bed 101 comprising at least one weight sensor 101b configured to detect the presence of a patient in the bed 101 and to generate a corresponding presence signal;
- a plurality of sensors 101c configured to detect the descending of the patient from the bed 101 and to generate a corresponding descending signal;
- at least one acoustic sensor 101e able to detect environmental noises near the bed 101 of the patient, to recognize words comprised into a specific dataset, and to generate a corresponding noise detection signal;
- at least one thermal camera 101d able to detect the environmental temperature near the bed 101 of the patient and the body temperature of the patient, and to generate a corresponding signal related to a danger concerning an excessive rise in the environmental temperature, near the patient, or in body temperature of the patient himself;
- at least one movement sensor 101f able to detect sudden movements of the patient in the bed 101, and to generate a corresponding alarm signal;
- at least one wearable sensor 102 placed on a garment or over the skin of the patient, configured to generate a corresponding fall and/or absence of the patient from a predetermined area alarm signal;
- at least one sensor 101g able to remotely monitor vital parameters of the patient, said vital parameters being comprised within: blood oxygen saturation; number of heartbeats; and arterial pressure value.

The safety system 100 comprises also:
- at least one embedded processing unit 101a, provided with a memory unit and with a local database, configured to acquire, filter and/or sample and save, to said local database, data related to the signals generated by the sensors 101b, 101c, 101e, 101d, 101f, 101g, 102;
- at least one computer 103, connected to the at least one embedded processing unit 101a, provided with an Internet access and configured to download the data, saved to the local database by the embedded processing unit 101a, through a wired or a wireless local network, and comprising a control system, a central database able to store the data related to the signals generated by the sensors 101b, 101c, 101e, 101d, 101f, 101g, 102 and/or entered by at least one healthcare professional, at least one graphic interface, specific for healthcare professionals, and a plurality of algorithms, for example machine learning algorithms, of logics of a different kind.

According to an aspect of the invention, such a plurality of algorithms is able to process the data saved to the central database, i.e., the data downloaded from each local database installed on a corresponding embedded processing unit 101a, related to each patient and his corresponding bed 101.

According to an aspect of the invention, the data entered by the at least one healthcare professional comprise data concerning the sleep-wake cycle, a pharmacological therapy and a diet followed by each patient.

According to an aspect of the invention, the plurality of algorithms comprised in the computer 103 is able to process also the data entered by the at least one healthcare professional, that comprise data concerning the sleep-wake cycle, the pharmacological therapy and the diet followed by each patient.

According to an aspect of the invention, the plurality of algorithms is able to generate and send visual and/or acoustic alarms and/or requests for preventive interventions to healthcare professionals, in particular to mobile terminals managed by said healthcare professionals.

According to an aspect of the invention, the plurality of algorithms is able to evaluate a degree of correlation between the temporal evolution of data related to patients having the same pathologies and generate graphs able to describe behavioral trends of the patients or trends concerning the pathologies under observation.

According to another aspect of the invention, the plurality of algorithms is able to evaluate, in addition, a further degree of correlation between the data concerning the sleep-wake cycle, the data related to the signal generated by the sensor 101g, the pharmacological therapy, the diet followed by each patient and the temporal evolution of the pathologies under observation.

Advantageously according to the invention, the measurement and evaluation of the aforementioned degree of correlation between the data concerning the sleep-wake cycle, the pharmacological therapy, the diet followed by each patient and the temporal evolution of the pathologies under observation, performed by means of supervised machine learning algorithms, allow to evaluate and verify the overall state of health of each patient, to correct potential deficiencies regarding therapy, diet or other parameters, and to determine potential cause-effect relationships between said parameters and the pathologies.

According to another aspect of the invention, the safety system 100 comprises also:
- a plurality of actuators configured for the remote control of appliances housed in the room, or environment, where the bed 101 is placed, for example the control of ambient lighting;
- at least one actuator configured for a remote night vision within the infrared range.

According to another aspect of the invention, the computer 103 queries, i.e., performs a polling, in asynchronous mode, of the at least one embedded processing unit 101a in order to download the data stored in the local database, or buffer, comprised in said embedded processing unit 101a.

According to another aspect of the invention, the data entered by health professionals in the central database comprised in the computer 103 relate to the intervention of said operators, following an alarm.

According to an aspect of the invention, the control system is configured to analyze the aforementioned data, related to the signals detected by the sensors 101b, 101c, 101e, 101d, 101f, 101g, 102, through the use of algorithms of different kind, and to generate a plurality of visual and/or acoustic alarms when the data received and analyzed, or a combination of said data, exceed the limits assigned to them and specific for each patient, with reference, for example, to the presence of the patient in the bed, when the values detected by the sensors exceed specific thresholds, but also in the case in which the combination of readings of several sensors corresponds to a pre-established dangerous condition for the patient considered in the environment, for example an abandonment of the bed, convulsive or uncontrolled movements in the bed, screams, incongruous movements in the environment, presence of fire or smoke, and/or even in the case in which there is a critical combination of the physical parameters monitored, concerning the patient himself. Furthermore, by applying machine learning algorithms in the analysis of data and trends saved to the central database, values of tuples of likelihood functions are generated in order to measure the evolution of a disease and/or of the behavior of the patient, with such values able to be used for the generation of requests for preventive interventions. In fact, the approximation of the values calculated for the likelihood functions of a specific patient in a given situation, to values detected and/or calculated in correspondence with previous and specific critical conditions, can numerically and preventively highlight the approaching of critical conditions which, in this way, can be prevented or mitigated. Even the approaching of the specific data detected, related to the patient and to the surrounding environment, or a combination of them, to values calculated with predetermined models for the type of disease of the patient considered, can generate the emission of an alarm signal, communicated for example remotely to an operator.

According to an aspect of the invention, the safety system 100 comprises at least one mobile terminal of a healthcare professional configured to remotely receive, from the computer 103, notifications concerning the aforementioned alarms and/or requests for preventive interventions, said alarms and said requests being able to be implemented by means of visual and/or acoustic signals.

According to an aspect of the invention, the safety system 100 includes at least one video camera, one microphone and one loudspeaker integrated in the embedded processing unit 101a, suitable for a remote verbal and visual interaction with the patient.

According to an aspect of the invention, the central database, installed on the computer 103, is configured for the creation and the management, starting from the acquired data, related to the patient and to the environment that hosts said patient, for example the room, of a dataset characterized by a plurality of records that allow to have a detailed and temporally congruent image of the state of the patient and of the environment hosting him, and also the evolution over time of the same data, of behavioral aspects of the patient and of his physiognomy, i.e., face/body over time.

According to an aspect of the invention, in said central database are saved, for each patient and for each pathology associated with the patient, a history of first data related to parameters associated with critical events of the patient himself, and second data concerning the application of models and algorithms to said history of the first data able to evaluate and predict, by means of correlation algorithms, the eventual exceeding of historical likelihood thresholds in the evolution of a predetermined pathology, and to generate corresponding alarms on the basis of the prediction/prevention functions of the safety system 100.

According to another aspect of the invention, the aforementioned threshold values are re-evaluated and updated based on the correlations and historical events for the same patient or for patients suffering from the same pathologies.

According to an aspect of the invention, advantageously, the safety system 100 comprises a plurality of models refined over time by means of the history of data and the application of machine learning algorithms to the same data, or models concerning the critical situations for each patient and for each associated pathology. The safety system 100, in fact, contemplates the creation of the history of the data in its database and, in particular, of the data associated with specific critical situations of each patient, together with the outcomes achieved from the application of the algorithms to the same data. The latter constitute the reference data for the subsequent evaluations of new data and, therefore, for the preventive determination of any new critical situations that may arise following the overcoming of the historical likelihood thresholds in the evolution of the disease under observation. Such threshold values are re-evaluated and updated in relation to the evolutions and historical events for the same patient or for patients suffering from the same pathologies and act as inputs, as described above, in the analysis of new data and trends acquired. Consequently, the safety system 100 provides not only an initial learning step for the machine learning algorithms, but also a constant learning and refinement of the inputs for the same algorithms, with reference to each patient.

According to an aspect of the invention, the safety system 100 comprises a plurality of behavioral models of the operators generated through the creation of a history of the interventions of the same operators, following the reception of alarm signals or requests for attention, and of the effects of the same interventions, also validated through feedback provided by the sensors/actuators, in order to identify the best assistance modalities to suggest in the future.

According to an aspect of the invention, the weight sensors 101b, configured for detecting the presence of the patient in the bed 101, are oleo dynamic weight sensors, installed in correspondence with an end portion of the bed 101, therefore at the foot of the bed 101, and are configured to evaluate the weight of the bed 101, with and without the patient present on the bed 101 itself.

Advantageously according to the invention, the use of weight sensors 101b of the oleo dynamic type allows a more accurate measurement of the weight of the bed 101 in the presence and absence of the patient, compared to weight sensors made with other technologies.

Advantageously, the safety system 100 is modular and scalable, therefore able to of be configured according to the specific characteristics of the person to be monitored, to the host environment, e.g., a nursing home, a retirement home, an isolated house and to the targets of monitoring and medical check.

A further advantage given by the use of the weight sensors 101b according to the invention, is that they can be used in any hospital or domestic bed.

Advantageously according to the invention, the acoustic sensor 101e allows to detect environmental noises and/or sounds emitted by the patient, whose sound intensity is higher than a preset threshold, with reference, for example, to screams.

Advantageously according to the invention, the machine learning algorithms running on the computer 103 are able to interpret automatically, by means of the acoustic sensor 101e, the words spoken by the patient within a predefined dataset of words, according to speech recognition logics.

According to an aspect of the invention, the embedded processing unit 101a is installed on the bed 101, for example in correspondence with one of its end portions, preferably at the footboard.

According to an aspect of the invention, the sensors 101c, 101e, 101d, 101f, 101e, 101g are installed in the embedded processing unit 101a.

Advantageously according to the invention, the use of the thermal camera 101d allows to promptly detect the presence of smoke or flames in proximity of the bed 101.

According to an aspect of the invention, the control system, running on the computer 103, associated on the same computer 103 with the machine learning algorithms developed on the basis of the behavioral analysis of the sampled and analyzed data, is able to process, through a comparison with known and/or developed models, the signals generated by the sensors 101b, 101c, 101e, 101d, 101f, 101g, 102 and by the video camera, generating immediate or preventive, visual and/or acoustic alarms, as previously mentioned.

Advantageously according to the invention, said machine learning algorithms allow to achieve a synergistic effect on the detections performed by all sensors, discriminating and correlating them in an intelligent way, minimizing the risk of generating false alarms and their corresponding notifications.

The safety system 100, through the plurality of sensors, in particular, composed as previously described, bases its behavior on the analysis of the events and is designed on the underlying machine learning logic, which is also able to discriminate false positives.

In detail such a logic, and the algorithms that supervise the behavior of the safety system 100 during its unsupervised learning, used to define a set of frequent behaviors in a given dataset, are preferably based on the "Apriori Machine Learning Algorithm", a simple and powerful data mining logic that generates an association rule on the transaction databases on which some workarounds are implemented, in order to improve its weak points. The extraction of association rules preferably uses Candidate-Generation-Based (CGB) and Pattern-Growth-Based strategies (PGB).

In particular, for sensors characterized by sudden TTL edges output such as the movement sensor, or tilt sensor, 101f, the algorithm calculates the number of pulses recorded during a predetermined time frame obtained from a series of events recorded in the database in a self-learning step, in order to determine whether the detected event is a true positive or a false positive.

In a similar way, although with different logics, the behavior of the sensors 101c configured to detect the descending of the patient from the bed 101, implemented for example through a lidar, is analyzed with regard to the detection times and the speed of displacements through the comparative analysis of the predictive models developed experimentally, also crossing the data coming from the weight sensors 101b of the oleo dynamic type; the algorithm is however based on a logic capable of self-adaptation in cases where too many false positives are recorded.

With regard to sensors having a linear analog output, such as the thermal camera 101d, the acoustic sensor 101e and the weight sensors 101b, specific functions that sample, through an ADC (Analog to Digital Converter) of a MCU (Micro Controller Unit) integrated in the embedded processing unit 101a, the measurements of the aforementioned sensors, at regular intervals and in a very short time, are performed.

According to another aspect of the invention, when the safety system 100 is applied to a plurality of beds 101, each embedded processing unit 101a is associated with a unique identification code, such a code allowing the association of any single bed 101 and of the hospital room with the registry of the related patient.

According to one aspect of the invention, the embedded processing unit 101a is equipped with a screen able to display images or videos.

Furthermore, according to another aspect of the invention, the embedded processing unit 101a allows to make and receive telephone and video calls, even without the intervention of the patient, by means of a self-call or a self-answer.

Advantageously according to the invention, the screen of the embedded processing unit 101a allows the patient to view images or videos that help him maintain a contact with his personal memories.

According to another aspect of the invention, each embedded processing unit 101a records the data acquired by itself on a local database, which hosts onboard, and an automatic mechanism guarantees the transfer and replication of the same records of the local database on a remote database available to the control system, followed by a simultaneous cancellation, on the embedded processing unit 101a, of the records already transferred.

Advantageously according to the invention, such a replication operation allows to store the data of any single patient safely, preventing at the same time any manipulation on the embedded processing unit 101a.

In addition, the architecture of the software modules is designed according to robustness and reliability criteria, through the historicization of each detected event, of each subsequent processing of each log and action of the operators on a remote server, while guaranteeing the integrity of the data and preventing their manipulation.

According to another aspect of the invention, and in particular for ambulatory patients, the wearable sensor 102 is a proximity sensor of the beacon type, not shown in the figure, coupled, by means of a short-range wireless standard, with the at least one embedded processing unit 101a, and is configured to detect and signal sudden accelerations to which the patient is subject and his presence within a predetermined area.

Advantageously according to the invention, through the measurements of the wearable sensor 102, the voluntary or involuntary exit of the patient from the room can be controlled, and the sudden accelerations to which the patient is subject can be detected and signaled.

According to an aspect of the invention, said proximity sensor 102 is connected through a short-range wireless standard to the embedded processing unit 101a. Furthermore, according to another aspect of the invention, the wearable sensor 102 is configured to detect and signal, to the embedded processing unit 101a, sudden accelerations to which the patient is subject by launching an SOS in the event of a fall, both accidental and due to fainting.

According to another aspect of the invention, the safety system 100 also comprises a software application with a web interface that allows to view from afar, through the Internet, the chronology of the visual and/or acoustic alarms and/or requests for preventive interventions generated by the control system running on the computer 103, on the basis of the signals coming from the sensors 101b, 101c, 101d, 101e, 101f, 101g, 102 and from the video camera, said alarms being sent by SMS, instant messaging or e-mail to portable devices such as smartphone or tablet managed by healthcare professionals.

According to another aspect of the invention, as previously described, said software application with a web interface is installed on the computer 103.

According to an aspect of the invention, such a web interface is able to configure, manage and monitor, and in particular consult, data concerning a plurality of rooms/patients to which the safety system 100 is applied, by consulting applications on fixed or portable devices such as PCs, tablets, smartphones, provided with specific user interfaces that allow operators to verify the status of patients and their rooms, receive alarm notifications or requests for attention, insertion of notes describing the performed interventions, the adjustment of alarm thresholds or attention ranges about certain situations that the main central application, i.e., the control system running on the computer 103 uses, while managing the overall system, as an input for the processing algorithms, customized for the needs of each patient.

According to another aspect of the invention, the embedded processing unit 101a and the computer 103 are connected by means of a wired or wireless LAN.

As mentioned, the present invention also refers to a safety method for monitoring not self-sufficient persons or patients with cognitive disorders, such a method comprising the steps of:
- detecting environmental noises near a bed of a patient whose sound intensity is higher than a preset threshold;
- automatically interpreting words spoken by the patient and comprised within a predefined dataset of words;
- detecting sudden movements in the bed where the patient is hospitalized;
- detecting the presence of flames and smoke near the bed;
- detecting an attempt of the patient to get out of the bed, both from the right side and from the left side of said bed;
- detecting the presence or the absence of the patient in the bed through the use of oleo dynamic weight sensors;
- detecting the presence/absence of the patient within a predetermined range of distances;
- detecting the vital parameters of the patient, said vital parameters being comprised within: body temperature, number of heartbeats, arterial pressure value, blood oxygen saturation;
- acquiring the signals generated during the aforementioned detection steps from, by means of an embedded processing unit positioned on the bed;
- having a computer, connected to said embedded processing unit, communicate with a device, such as a tablet, provided with a software application having a web interface and configured to display the history of visual and acoustic alarms generated by said computer, following the aforementioned detection steps.

In particular, the safety method for monitoring not self-sufficient persons by means of the safety system 100, comprises the steps of:
- detecting the presence or the absence of a patient in a bed using oleo dynamic weight sensors, sudden movements of the patient in the of the bed, the presence of flames and smoke near the bed, the temperature of the patient, attempts to get out of the bed made by the patient, the presence of the patient within a predetermined distance from the bed and/or a fall of said patient, vital parameters of the patient, noises above predefined thresholds, detection and recognition of words within a specific dataset, and generating corresponding signals;
- acquiring the signals detected in the previous step, by means of an embedded processing unit mounted on the bed;
- providing a connection between the embedded processing unit and a computer provided with a software application having a web interface;
- performing and visualizing, by means of said computer, a historical record of the visual and acoustic alarms generated by the embedded processing unit during the previous detection step.

According to an aspect of the invention, each alarm and/or request for preventive interventions is uniquely associated with a patient and a room and may also be notified to and displayed by a mobile terminal of a remote user, for example a healthcare professional provided with a smartphone or a tablet, by means of an SMS, instant messaging or an e-mail.

According to another aspect of the invention, the method for monitoring not self-sufficient persons comprises the activation of a full-duplex video and acoustic connection to the patient, following the receipt of an alarm also on the mobile terminal, in order to monitor the status of the patient and interact with the patient himself.

According to one aspect of the invention, the healthcare professional, following a notification of an alarm, can observe the patient through the video camera, interacting with the patient himself.

Furthermore, advantageously according to the invention, by using the safety system and the method described, a healthcare professional is able to resolve the alarm concerning a patient by approaching the same patient and, subsequently, by recording the resolution of the issue of one or more patients, uniquely identified, going with certainty to the room of the patient from which the alarm has been generated, thus carrying out the necessary check.

Furthermore, advantageously according to the invention, the health care professional is able to:
- manage the alarm by reporting and recording the date, time and way of resolution;
- reset potential malfunctions or set one or more peripherals that might have caused false alarms by modifying the concerning threshold or disabling a peripheral in case of failure;
- consult the history of the alarms received, filtering said alarms by the name pf the patient.

Therefore, the safety system and method allow to monitor patients from afar.

In addition, the safety system and method for monitoring patients not self-sufficient or with cognitive disorders are efficient.

Finally, the safety system and method allow to perform said monitoring in real-time, in a reliable, fast and efficient way, optimizing the human resources working both in the clinic and in the family, reducing the so-called "caregiver effort".

It is finally clear that the safety system and method for monitoring not self-sufficient persons, described and illustrated herein, can be subject to modifications and variations without departing from the scope of the present invention, as defined in the appended claims.

## Claims

1. Safety system (100) for monitoring not self-sufficient persons, comprising:
- at least one bed (101) comprising at least one weight sensor (101b) configured to detect the presence of a patient in the bed (101) and to generate a corresponding presence signal;
- a plurality of sensors (101c) configured to detect the descending of the patient from the at least one bed (101) and to generate a corresponding descending signal;
- at least one acoustic sensor (101e) able to detect environmental noises near the at least one bed (101) of the patient, to recognize words comprised into a specific data set, and to generate a corresponding noise detection signal;
- at least one thermal camera (101d) able to detect the environmental temperature near the at least one bed (101) of the patient and the temperature of the patient, and to generate a corresponding signal related to a danger of an excessive rise in the environmental temperature or the temperature of the patient;
- at least one movement sensor (101f) able to detect sudden movements of the patient in the at least one bed (101), and to generate a corresponding alarm signal;
- at least one wearable sensor (102) placed on a garment or over the skin of the patient, configured to generate a corresponding fall and/or absence of the patient from a predetermined area alarm signal;
- at least one embedded processing unit (101a), provided with a memory unit and with a local database, configured to acquire, filter and/or sample and save to said local database data related to the signals generated by the sensors (101b, 101c, 101e, 101d, 101f, 102);
- at least one computer (103), connected to the at least one embedded processing unit (101a), provided with an Internet access, comprising a control system, a central database comprising data entered by at least one healthcare professional and at least one specific graphic interface for healthcare professional, said at least one computer (103) being configured to download to said central database, by means of a wired or wireless local network, the data saved to the local database;
- at least one mobile terminal of the at least one healthcare professional;
- at least one video camera, one microphone and one loudspeaker mounted on the at least one embedded processing unit (101a) and configured for a vocal and visual remote interaction with the patient;
**characterized in** comprising at least one sensor (101g) able to remotely monitor vital parameters of the patient, said vital parameters being comprised within: blood oxygen saturation; number of heartbeats; and arterial pressure value, **in that** the data entered by the at least one healthcare professional comprise data concerning a sleep-wake cycle, a pharmacological therapy and a diet followed by each patient, and **in that** the at least one computer (103) comprises a plurality of algorithms configured to process the data saved to the central database and data related to a signal generated by the at least one sensor (101g), to generate and send visual and/or acoustic alarms and/or requests for preventive interventions to the mobile terminal of the at least one health care professional, to evaluate a degree of correlation between the temporal evolution of data related to patients having the same pathologies, to evaluate a degree of correlation between the data concerning the sleep-wake cycle, the data related to the signal generated by the sensor (101g), the pharmacological therapy, the diet followed by each patient and the temporal evolution of the pathologies under observation, in order to evaluate the overall state of health of each patient, and to generate graphs able to describe behavioral trends of the patients or trends concerning the pathologies under observation.

2. Safety system (100) according to claim 1, **characterized in that** the weight sensors (101b) are oleo dynamic sensors and are installed in correspondence with an end portion of the bed (101).

3. Safety system (100) according to claim 1, **characterized in that** the at least one embedded processing unit (101a) is placed at an end portion of the bed (101) and houses the sensors (101c, 101e, 101d, 101f) and the video camera, said at least one embedded processing unit (101a) comprising a display configured for viewing images and being configured for making and receiving phone calls and video calls.

4. Safety system (100) according to claim 1, **characterized in that** the least one bed (101) is associated with a unique identification code and **in that** said at least one embedded processing unit (101a) is associated with the unique identification code of the at least one bed (101) and of a hospital room of the patient where the at least one bed (101) is placed.

5. Safety system (100) according to claim 1, **characterized in** comprising:
- a plurality of actuators configured for the remote control of appliances housed in the room where the bed is placed (101);
- at least one actuator configured for a remote night vision within the infrared range.

6. Safety system (100) according to claim 1, **characterized in that** the at least one wearable sensor (102) is a proximity beacon sensor coupled through a short-range wireless connection with the at least one embedded processing unit (101a), and is configured for detecting and notifying sudden accelerations to which the patient is subject and the presence of said patient within a prefixed area.

7. Safety system (100) according to claim 1, **characterized in** comprising a software program provided with a web interface installed on the computer (103) and configured to show a historical record of the visual and/or acoustic alarms and/or requests for preventive interventions generated by the control system running on the at least one computer (103).

8. Safety system (100) according to claim 1, **characterized in that** to said central database is saved for each patient and for each pathology associated with said patient, a historical record of first data related to parameters associated with critical events concerning the patient, and second data related to the application of models and algorithms to the historical record of the first data able to evaluate and predict, by means of correlation algorithms, the eventual overcoming of historicized likelihood thresholds concerning the evolution of a predetermined pathology and to generate corresponding alarms, said threshold values being re-evaluated and updated on the basis of correlations and historicized events and related the same patient or for patients affected by the same pathologies.

9. Safety method for monitoring not self-sufficient persons by means of the safety system (100) according to one of the previous claims, comprising the steps of:
- detecting the presence or the absence of a patient in a bed using oleo dynamic weight sensors, sudden movements of the patient in the of the bed, the presence of flames and smoke near the bed, the temperature of the patient, attempts to get out of the bed made by the patient, the presence of the patient within a predetermined distance from the bed and/or a fall of said patient, vital signs of the patient, noises above predefined thresholds, detection and recognition of words within a specific dataset, and generating corresponding signals;
- acquiring signals detected during the previous step by means of an embedded processing unit mounted on the bed;
- providing a connection between the embedded processing unit and a computer provided with a software program provided with a web interface;
- performing and visualizing, by means of said computer, a historical record of the visual and acoustic alarms generated by the embedded processing unit during the previous detection step.

10. Safety method for monitoring not self-sufficient persons according to claim 9, **characterized in that** each alarm and/or request for preventive interventions is uniquely associated with a patient and with a room, and is able to be notified to and displayed by a mobile terminal of a remote user by means of an SMS, instant messaging or an e-mail.
